# EUROPÄISCHE PATENTSCHRIFT

(11) **EP 1 337 227 B1**
(45) Veröffentlichungstag und Bekanntmachung des Hinweises auf die Patenterteilung: **14.06.2006**
(21) Anmeldenummer: 01998314.7
(22) Anmeldetag: 21.11.2001
(51) Int. Cl.: A61K 8/34, A61K 8/92, A61Q 15/00, A61Q 19/00, A61Q 19/10

(54) **VERWENDUNG VON NANOPARTIKULÄREN WACHSEN IN DER HAUTKOSMETIK**
USE OF NANOPARTICULATE WAX IN SKIN COSMETICS
UTILISATION DE CIRES NANOPARTICULAIRES EN COSMETIQUE POUR LA PEAU

(30) Priorität: 01.12.2000 DE 10059668
(43) Veröffentlichungstag der Anmeldung: 27.08.2003
(73) Patentinhaber: Henkel Kommanditgesellschaft auf Aktien, 40589 Düsseldorf (DE)
(72) Erfinder: HUNDEIKER, Claudia, 40549 Düsseldorf (DE); KROPF, Christian, 40721 Hilden (DE); SCHULZE ZUR WIESCHE, Erik, 20251 Hamburg (DE); SCHRÖDER, Christine, 40593 Düsseldorf (DE); HOLLENBROCK, Martina, 40723 Hilden (DE); FÖRSTER, Thomas, 40699 Erkrath (DE); DOLHAINE, Hans, 41352 Korschenbroich (DE)
(86) Internationale Anmeldenummer: PCT/EP2001/013483
(87) Internationale Veröffentlichungsnummer: WO 2002/043673

(56) Entgegenhaltungen:
- EP-A- 0 506 197
- WO-A-00/10522
- FR-A- 2 666 015

## Beschreibung

Die Erfindung betrifft die Verwendung nanopartikulärer Wachse, wobei das Wachs einen Schmelzpunkt von mindestens 40°C hat und wobei der mittlere Teilchendurchmesser im Bereich von 5 bis 500 nm liegt, zur Herstellung von kosmetischen und/oder dermatologischen Zubereitungen und zur Pflege der menschlichen Haut und/oder der Nägel.

Die Verwendung von Wachsen in der Kosmetik ist lange bekannt. So werden Wachse nicht nur als Rezepturgrundlage und Konsistenzregler verwendet, sondern auch als Emollientien zur Verbesserung der Geschmeidigkeit der Haut. Um das Wachs in ausreichender Konzentration formulieren zu können, werden in der Regel flüssige bis halbfeste Zubereitungen verwendet, welche verschiedene Nachteile haben. Dazu zählen eine schmierige Konsistenz der Formulierung, ein Glänzen von behandelten Hautpartien, sowie die Fleckenbildung auf Textilien. Wachse lassen sich zudem häufig in kosmetischen Zubereitungen nur unzureichend dispergieren, so daß keine hinreichende Konzentration an Wachs in der Zubereitung erreicht wird, um die bei der Anwendung gewünschten kosmetischen Effekte des Wachses zu erzielen. Andere aus dem Stand der Technik bekannte wachshaltige kosmetische Formulierungen sind nicht ausreichend stabil, so daß es zu Phasentrennungen oder zur Sedimentation von Wachspartikeln kommt. Diese Probleme treten beispielsweise häufig bei sogenannten Rinse-off-Formulierungen auf, welche wäßrig basiert sind und nach der Anwendung von der Haut abgespült werden.
Die Verwendung von Wachsen in schäumenden Formulierungen wird zudem dadurch limitiert, daß stark lipophile Stoffe wie Wachse schaumdämpfend wirken.

Dadurch lassen sich Wachse für viele Anwendungen nicht im gewünschten Ausmaß in schäumende Formulierungen einarbeiten.

In der europäischen Patentschrift EP-B1 0 506 197 sind wäßrige Suspensionen von Lipoidnanoteilchen beschrieben, welche aus mindestens einem Lipid und vorzugsweise auch mindestens einem Emulgator bestehen und eine Teilchengröße zwischen 50 und 1000 nm haben. Die Suspensionen dienen zur Herstellung von Zusammensetzungen für die topische Behandlung der Haut, der Haare und der Nägel.

WO 00/10522 A1 offenbart wässrige Dispersionen nanopartikulärer Wachse mit einem Teilchendurchmesser unter 500 nm, die mit Ethylenoxidanlagerungsprodukten an Fettalkohole mit 16 - 22 C-Atomen und an Fettsäuremonoglyceride oder an Sorbitanmonoester von Fettsäuren mit 16 - 22 C-Atomen stabilisiert sind, sowie deren Verwendung in kosmetischen Zusammensetzungen.

EP 506 197 A1 offenbart wässrige Dispersionen nanopartikulärer Wachse, die mit diversen Emulgatoren (kationische anionische und amphotere Emulgatoren, weiterhin Polyoxyethylenpolyoxypropylenalkylether, Polyoxyethylenfettsäureester, Polyoxyethylensorbitanfettsäureester, Polyoxyethylenalkylether, Sorbitanester, Sucroseester, Lecithine, Siliconemulgatoren, Betaine und Polyglycerylfettsäureester) stabilisiert sind, sowie deren Verwendung in topischen Zusammensetzungen.

FR 2 666 015 A1 offenbart kosmetische und/oder dermopharmazeutische Zusammensetzungen, die eine wässrige Dispersion nanopartikulärer Wachse mit einer Teilchengröße kleiner 500 nm, die mit diversen anionischen oder nicht-ionischen Emulgatoren stabilisiert sind, sowie deren Verwendung zur Verabreichung von Retinoiden in die Haut.

Aufgabe der vorliegenden Erfindung war es, die Verwendungsmöglichkeiten für Wachse in hautkosmetischen Zubereitungen zu erweitern und Zubereitungen mit verbesserter Pflegewirkung auf die Haut zur Verfügung zu stellen.

Es wurde gefunden, daß sich nanopartikuläre Wachse mit einem Schmelzpunkt von mindestens 40°C und einem mittleren Teilchendurchmesser des Wachses im Bereich von 5 bis 500 nm und vorzugsweise im Bereich von 10 bis 200 nm in besonderem Maße zur Herstellung von kosmetischen und/oder dermatologischen Zubereitungen eignen und daß sie aufgrund ihrer Wirkungen besonders zur kosmetischen Pflege der menschlichen Haut und/oder der Nägel geeignet sind.

Gegenstand der Erfindung ist eines nanopartikuläres Wachses, wobei das Wachs einen Schmelzpunkt von mindestens 40°C hat, der mittlere Teilchendurchmesser des Wachses im Bereich von 5 bis 200 nm liegt, und von mindestens einem Oberflächenmodifikationsmittel, das ein Alkylpolyglycosid ist, umhüllt ist.

Ein weiterer Gegenstand der Erfindung ist eine kosmetische oder dermatologische Zubereitung, enthaltend ein oder mehrere übliche Adjuvantien, ausgewählt aus Konservierungsmitteln, Bakteriziden, Antioxidantien, Parfüms, Schaumbremsen, Farbstoffen, Pigmenten, Verdickungsmitteln, anfeuchtenden und/oder feuchthaltenden Substanzen, Tensiden, Emulgatoren, Weichmachern, Fetten, Ölen, Wachsen, Silikonen, Sequestrierungsmitteln, anionischen, kationischen, nichtionischen oder amphoteren Polymeren, Alkalinisierungs- oder Acidifizierungsmitteln, Alkoholen, Polyolen, Enthärtern, Lichtschutzmitteln, Elektrolyten und organischen Lösungsmitteln, sowie weiterhin ein nanopartikuläres Wachs, wobei das Wachs einen Schmelzpunkt von mindestens 40°C hat, der mittlere Teilchendurchmesser des Wachses im Bereich von 5 bis 200 nm liegt, und das Wachs von mindestens einem Oberflächenmodifikationsmittel, das ein Alkylpolyglycosid ist, umhüllt ist.

Ein weiterer Gegenstand der Erfindung ist die nicht-therapeutische, kosmetische Verwendung eines nanopartikulären Wachses, wobei das Wachs einen Schmelzpunkt von mindestens 40°C hat, der mittlere Teilchendurchmesser des Wachses im Bereich von 5 bis 200 nm liegt, und das Wachs von mindestens einem Oberflächenmodifikationsmittel das ein Alkylpolyglycosid ist, umhüllt ist, zur Reinigung und/oder Pflege der menschlichen Haut und/oder der Nägel.

Die Größenangaben sind zu verstehen als Durchmesser in Richtung der größten Längenausdehnung der Teilchen. Bei der Herstellung der feinteiligen Partikel erhält man stets Teilchen mit einer Größe, die einer Verteilungskurve folgt. Zur experimentellen Bestimmung der Teilchengröße kann beispielsweise die dem Fachmann bekannte Methode der dynamischen Lichtstreuung angewandt werden.

Unter Wachsen sind im Sinne der Erfindung natürliche oder synthetische Stoffe zu verstehen, welche bei 20°C knetbar, fest bis brüchig hart, grob bis feinkristallin, durchscheinend bis opak, jedoch nicht glasartig sind, oberhalb von ca. 40°C unzersetzt schmelzen und schon wenig oberhalb des Schmelzpunktes niedrigviskos und nicht fadenziehend sind. Die im Sinne der Erfindung einzusetzenden Wachse unterscheiden sich beispielsweise von Harzen dadurch, daß sie in der Regel etwa zwischen und 50 und 90°C, in Ausnahmefällen auch bis zu 200°C, in den schmelzflüssigen, niedrigviskosen Zustand übergehen und praktisch frei von aschebildenden Verbindungen sind. Nach ihrer Herkunft teilt man die Wachse in die folgenden drei Gruppen ein: natürliche Wachse pflanzlichen oder tierischen Ursprungs, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse oder hydriertes Rizinusöl sowie synthetische Wachse, wie z.B. Polyalkylenwachse und Polyethylenglycolwachse. Ein erfindungsgemäß besonders bevorzugtes Wachs ist hydriertes Rizinusöl.

Ebenso sind zu den Wachsen im Sinne der Erfindung die höheren Fettalkohole sowie die Wachsester zu zählen, sofern sie einen Schmelzpunkt von mindestens 40°C haben.

Unter höheren Fettalkoholen sind primäre aliphatische Alkohole der Formel (I) zu verstehen,

R¹OH (I)

in der R¹ für einen aliphatischen, linearen oder verzweigten Kohlenwasserstoffrest mit 14 bis 40 Kohlenstoffatomen und 0, 1, 2 oder 3 Doppelbindungen steht. Typische Beispiele sind Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol, Brassidylalkohol, Lignocerylalkohol, Cerylalkohol, Myricylalkohol, Melissylalkohol sowie deren Mischungen. Die Fettalkohole können pflanzlichen, tierischen oder synthetischen Ursprungs sein. Erfindungsgemäß verwendbare synthetische Fettalkohol-Gemische werden z. B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen erhalten. Bevorzugt sind höhere Fettalkohole pflanzlichen Ursprungs, besonders bevorzugt solche mit 16 bis 18 Kohlenstoffatomen. Weiterhin bevorzugt ist Cetylstearylalkohol, worunter Gemische aus etwa gleichen Teilen Cetyl- und Stearylalkohol zu verstehen sind.

Unter Wachsestern sind Stoffe zu verstehen, die der Formel (II) folgen,

R¹COO-R² (II)

in der R¹CO für einen linearen oder verzweigten Acylrest mit 6 bis 22 Kohlenstoffatomen, mit 0 bis 3 Doppelbindungen und mit 0 bis 3 Hydroxy-Substituenten steht und R² für einen linearen oder verzweigten Alkyl- und/oder Alkenylrest mit 6 bis 22 Kohlenstoffatomen, mit der Maßgabe, daß die Anzahl der Kohlenstoffatome im Ester mindestens 20 beträgt. Typische Beispiele hierfür sind Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat, Erucylerucat sowie Ester der Ricinolsäure. Erfindungsgemäß bevorzugt sind Wachsester, in welchen R¹CO für einen linearen Acylrest mit 16 oder 18 Kohlenstoffatomen steht.

Die nanopartikulären Wachspartikel im Sinne der vorliegenden Erfindung sind vorzugsweise von mindestens einem Oberflächenmodifikationsmittel ummantelt, wobei es sich im Sinne der Erfindung versteht, daß auch die ummantelten Wachs-Nanopartikel einen Schmelzpunkt von mindestens ca. 40°C haben.

Unter Oberflächenmodifikationsmitteln sind Stoffe zu verstehen, welche der Oberfläche der Nanopartikel physikalisch anhaften, mit diesen jedoch vorzugsweise nicht chemisch reagieren. Die einzelnen an der Oberfläche adsorbierten Moleküle der Oberflächenmodifikationsmittel sind im wesentlichen frei von intermolekularen Bindungen untereinander. Unter Oberflächenmodifikationsmitteln sind insbesondere Dispergiermittel zu verstehen. Dispergiermittel sind dem Fachmann beispielsweise auch unter den Begriffen Emulgatoren, Schutzkolloide, Netzmittel und Detergentien bekannt.

Als Oberflächenmodifikationsmittel kommen beispielsweise Emulgatoren vom Typ der nichtionischen Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipenta-erythrit, Zuckeralkohole (z.B. Sorbit), Alkylglucoside (z.B. Methylglucosid, Butylglucosid, Lauryl-glucosid) sowie Polyglucoside (z.B. Cellulose);
(9) Mono-, Di- und Trialkylphosphate sowie Mono-, Di- und/oder Tri-PEG-alkylphosphate und deren Salze;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß DE-PS 1165574 und/oder Mischester von Fettsäuren mit 6 bis 22 Kohlenstoffatomen, Methylglucose und Polyolen, vorzugsweise Glycerin oder Polyglycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für anionische Tenside und Emulgatoren sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate wie beispielsweise Fettalkoholethersulfate, Glycerinethersulfate, Hydroxymischethersulfate, Monoglycerid(ether)sulfate, Fettsäureamid(ether)sulfate, Mono- und Dialkyl-sulfosuccinate, Mono- und Dialkylsulfo-succinamate, Sulfotriglyceride, Amidseifen, Ethercarbonsäuren und deren Salze, Fettsäureisethionate, Fettsäuresarcosinate, Fettsäuretauride, N-Acylaminosäuren wie beispielsweise Acylglutamate und Acylaspartate, Alkyloligoglucosidsulfate, Proteinfettsäurekondensate (insbesondere pflanzliche Produkte auf Weizenbasis), und Alkyl(ether)-phosphate. Sofern die anionischen Tenside Polyglycoletherketten enthalten, können diese eine konventionelle, vorzugsweise jedoch eine eingeengte Homologenverteilung aufweisen.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammonium-glycinat, N-Acylamino-propyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methyl-quaternierte Difettsäuretriethanolaminester-Salze, besonders bevorzugt sind.

Als Oberflächenmodifikationsmittel geeignete Schutzkolloide sind z.B. natürliche wasserlösliche Polymere wie z. B. Gelatine, Casein, Gummi arabicum, Lysalbinsäure, Stärke, Albumin, Alginsäure sowie deren Alkali- und Erdalkalimetallsalze, wasserlösliche Derivate von wasserunlöslichen polymeren Naturstoffen wie z. B. Celluloseether wie Methylcellulose, Hydroxyethylcellulose, Carboxymethylcellulose oder modifizierte Carboxymethyl-cellulose, Hydroxyethyl-Stärke oder Hydroxypropyl-Guar, sowie synthetische wasserlösliche Polymere, wie z. B. Polyvinylalkohole, Polyvinylbutyrale, Polyvinylpyrrolidone, Polyalkylenglycole, Polyasparaginsäuren und Polyacrylate.

Als Oberflächenmodifikationsmittel bevorzugt sind Gelatine, Stärke, Dextrin, Dextran, Pektin, Gummi arabicum, Kasein, Polyvinylalkohole, Polyvinylbutyrale, Polyvinylpyrrolidone, Polyalkylenglycole, Methylcellulose, Carboxymethylcellulose, Hydroxypropylcellulose, Fettalkoholpolyglykolether, Fettalkoholpolyglycoside, Fettsäurealkanolamide, Glycerolester und Sorbitanester. Besonders bevorzugt sind Alkylpolyglycoside, in welchen der Alkylrest zwischen 12 und 16 Kohlenstoffatome enthält.

Die Menge des Oberflächenmodifikationsmittels in Bezug auf das Wachs entspricht mindestens der Menge, die erforderlich ist, um eine stabile Dispersion an Wachs-Nanopartikeln zu erhalten. Diese minimale Menge kann jeweils durch einfache Routineversuche ermittelt werden.

In der Regel wird für die Herstellung des nanopartikulären Wachses das Wachs und das Oberflächenmodifikationsmittel im Gewichtsverhältnis 1 : 50 bis 50 : 1, vorzugsweise 1 : 5 bis 10 : 1 und insbesondere 1 : 2 bis 2 : 1 eingesetzt. Anstelle der angegebenen Mengenanteile des Oberflächenmodifikationsmittels kann auch in gleicher Menge ein Gemisch aus zwei oder mehr Oberflächenmodifikationsmitteln eingesetzt werden.

Die erfindungsgemäß verwendeten nanopartikulären Wachsteilchen können nach unterschiedlichen Verfahren hergestellt werden.

Bei der Herstellung nach dem Evaporationsverfahren wird das Wachs zunächst in einem geeigneten organischen Lösungsmittel, wie z. B. einem Alkan, verzehrbaren Öl, Alkohol, Ether, Ester, Keton oder Acetal gelöst. Anschließend wird diese Lösung derart in ein Nicht-Lösungsmittel für das Wachs, vorzugsweise Wasser, einen niedrigen Alkohol oder Gemische aus mehreren Nicht-Lösungsmitteln, gegebenenfalls in Gegenwart eines darin gelösten Oberflächenmodifikationsmittels, gegeben, daß es durch die Vermischung von organischem Lösungsmittel und Nicht-Lösungsmittel zu einer Ausfällung des nanopartikulären Wachses kommt. Diese Vermischung erfolgt bevorzugt auf eine Weise, bei der das organische Lösungsmittel verdampft und weitgehend aus dem System abgetrennt wird. Das Lösungsmittel kann aber auch nach der Vermischung z. B. durch Destillation entfernt werden. Anstelle der Lösung aus Wachs und organischem Lösungsmittel kann auch eine O/W-Emulsion oder O/W-Mikroemulsion eingesetzt werden, in der die Lösung die Ölkomponente bildet. Als oberflächenaktive Verbindungen können die bereits eingangs erläuterten Oberflächenmodifikationsmittel verwendet werden.

Eine weitere Möglichkeit zur Herstellung der nanopartikulären Teilchen besteht in dem sogenannten GAS-Verfahren (Gas Anti Solvent Recrystallization). Das GAS-Verfahren nutzt ein hochkomprimiertes Gas oder überkritisches Fluid (z.B. Kohlendioxid) als Nicht-Lösungsmittel zur Kristallisation von gelösten Stoffen. Die verdichtete Gasphase wird in die Lösung des Wachses in einem geeigneten organischen Lösungsmittel, wie vorstehend beschrieben, eingeleitet und dort absorbiert. Dabei vergrößert sich das Flüssigkeitsvolumen, die Löslichkeit des Wachses nimmt ab und es wird in Form feinteiliger Partikel abgeschieden.

Weiter geeignet ist das PCA-Verfahren (Precipitation with a Compressed Fluid Anti-Solvent). Hier wird die Lösung des Wachses in einem geeigneten organischen Lösungsmittel, wie vorstehend beschrieben, in ein überkritisches Fluid, wie beispielsweise Kohlendioxid, eingedüst. Dabei bilden sich feinstverteilte Tröpfchen, in denen Diffusionsvorgänge ablaufen, die zu einer Ausfällung feinster Partikel führen.

Beim PGSS-Verfahren (Particles from Gas Saturated Solutions) wird das Wachs durch Aufpressen von Gas (z. B. Kohlendioxid oder Propan) aufgeschmolzen. Druck und Temperatur erreichen nahe- oder überkritische Werte. Die Gasphase löst sich im Feststoff und bewirkt eine Absenkung der Schmelztemperatur, der Viskosität und der Oberflächenspannung. Bei der Expansion durch eine Düse kommt es durch Abkühlungseffekte zur Bildung feiner Teilchen.

Beim RESS-Verfahren (Rapid expansion of supercritical solutions) wird das Wachs in einem Druckgefäß vorgelegt, der Reaktor verschlossen und das überkritische Lösungsmittel (CO₂ Ethylen, Propan, Ammoniak, Methanol, N₂O, N₂O₂, SF₆, Difluormethan, Trifluormethan, Wasser, Toluol, etc., bevorzugt jedoch CO₂) solange unter Temperaturerhöhung aufgepreßt, bis die gewünschten überkritischen Bedingungen erreicht sind. Zusammen mit dem Wachs kann auch ein Oberflächenmodifikationsmittel vorgelegt werden. Nachdem das Wachs, ggf. in Gegenwart des Oberflächenmodifikationsmittels, unter überkritischen Bedingungen gelöst wurde, wird die Lösung durch eine Düse expandiert, wobei es zur Bildung von Nanopartikeln kommt. Die Expansion kann in Luft, ein anderes Gas oder aber auch in eine Flüssigkeit, z.B. Wasser, hinein erfolgen, wobei diese Flüssigkeit wiederum ein Oberflächenmodifikationsmittel enthalten kann, um ein unerwünschtes Zusammenbacken der Partikel zu verhindern.

Beim Schmelz-Emulgier-Verfahren wird das Wachs in eine Flüssigkeit gebracht, in der es nicht löslich ist (Nicht-Lösungsmittel). Als Nicht-Lösungsmittel kommen Wasser oder Mischungen aus Wasser und nichtwässrigen polaren Lösungsmitteln, wie beispielsweise Ethanol, Glycerin, Ethylenglykol, Propylenglykol oder N-Methylpyrrolidon in Betracht. Als Nicht-Lösungsmittel bevorzugt ist Wasser. Anschließend wird das Gemisch über den Schmelzpunkt des Wachses unter Ausbildung eines Zweiphasensystems erhitzt. Durch Zugabe eines oder mehrerer Oberflächenmodifikationsmittel wird die flüssige Phase des geschmolzenen Wachses emulgiert, und zwar so, daß sehr feine Tröpfchen entstehen. Die Emulgierung erfolgt unter Einbringung von mechanischer Energie mittels eines herkömmlichen Rührers, beispielsweise eines Blatt- oder Ankerrührers. Nach der Bildung der Mikroemulsion wird diese unter den Schmelzpunkt des Wachses abgekühlt, wobei eine Nano-Dispersion des Wachses entsteht. Das Oberflächenmodifikationsmittel kann auch bereits zu Beginn des Verfahrens vorgelegt werden, bevor das Wachs geschmolzen wird. In einer weiteren Abwandlung des Verfahrens wird das Wachs aufgeschmolzen, ggf. in Gegenwart eines Oberflächenmodifikationsmittels. Davon getrennt wird das Nicht-Lösungsmittel auf eine Temperatur oberhalb des Schmelzpunkts des Wachses erwärmt, ggf. in Gegenwart eines Oberflächenmodifikationsmittels. Anschließend wird die Schmelze des Wachses, ggf. im Gemisch mit dem Oberflächenmodifikationsmittel, in das erwärmte Nicht-Lösungsmittel unter Rühren eingetropft und nach erfolgter Emulgierung unter den Schmelzpunkt des Wachses abgekühlt.

Bei der Herstellung von im Rahmen der vorliegenden Erfindung verwendeten nanopartikulären Wachs-Dispersionen wurde überraschenderweise gefunden, daß im Gegensatz zu der aus dem Stand der Technik bekannten Lehre, beispielsweise derjenigen des europäischen Patentes 0 506 197, eine intensive Durchmischung des Reaktionsgemischs während der Emulgierung des Wachses nicht erforderlich ist. Spezielle Vermischungstechniken wie die Verwendung von Hochgeschwindigkeitsrührem oder Hochdruckhomogenisatoren oder die Anwendung von Ultraschall sind demnach nach dem im Rahmen der vorliegenden Erfindung gefundenen Verfahren nicht erforderlich. Dies bedeutet für die technische Durchführung des Verfahrens einen erheblichen wirtschaftlichen Vorteil.

Im Rahmen der vorliegenden Erfindung wurde somit ein Verfahren zur Herstellung wäßriger Dispersionen von nanopartikulären Wachsen gefunden, wobei das Wachs einen Schmelzpunkt von mindestens 40°C hat und wobei der mittlere Teilchendurchmesser des Wachses in der Dispersion im Bereich von 5 bis 500 nm und vorzugsweise im Bereich von 10 bis 200 nm liegt, in welchem
(a) das geschmolzene Wachs in Gegenwart von Wasser mittels eines oder mehrerer Oberflächenmodifikationsmittel emulgiert wird,
(b) wobei die Emulgierung ohne intensive Durchmischung, insbesondere ohne Anwendung eines Hochgeschwindigkeitsrührers oder Hochdruckhomogenisators oder vergleichbarer Durchmischungsvorrichtungen erfolgt, und
(c) die Emulsion anschließend unter den Schmelzpunkt der emulgierten Wachströpfchen abgekühlt wird.

Als Oberflächenmodifikationsmittel eignen sich besonders nichtionische Emulgatoren, wobei Alkylpolyglycoside, insbesondere solche, deren Alkylrest 12 bis 16 Kohlenstoffatome enthält, bevorzugt sind.

Ein weiterer Gegenstand der Erfindung ist somit ein nanopartikuläres Wachs, wobei das Wachs einen Schmelzpunkt von mindestens 40°C hat und wobei der mittlere Teilchendurchmesser des Wachses im Bereich von 5 bis 500 nm und vorzugsweise im Bereich von 10 bis 200 nm liegt, welches herstellbar ist durch
(a) Emulgierung des geschmolzenen Wachses in Gegenwart von Wasser mittels eines oder mehrerer Oberflächenmodifikationsmittel, ohne daß die Emulgierung unter intensiver Durchmischung erfolgt, insbesondere ohne daß ein Hochgeschwindigkeitsrührer oder Hochdruckhomogenisator verwendet wird, und
(b) Abkühlung der Emulsion unter den Schmelzpunkt der emulgierten Wachströpfchen.

Die nach dem erfindungsgemäßen Verfahren hergestellten wäßrigen Dispersionen nanopartikulärer Wachse zeichnen sich durch eine hohe Lagerstabilität aus.

Aus der EP-A2 0 786 251 ist beispielsweise ein Verfahren zur Herstellung einer Dispersion von hydriertem Rizinusöl bekannt, in welchem hydriertes Rizinusöl mit Wasser in Gegenwart von Natrium-Laurylsulfat unter Anwendung eines Hochdruckhomogenisators dispergiert wird. Nach dem Abkühlen der gebildeten Dispersion wird zunächst eine Dispersion mit einer mittleren Teilchengröße von 195 nm erhalten, die sich nach 24 Stunden zu einem Gel verfestigte. Für kosmetische Verwendungen im Sinne der vorliegenden Erfindung ist jedoch ein derartiges Gel nicht bzw.nur eingeschränkt einsetzbar. Nach dem Verfahren der vorliegenden Erfindung wurde mit Alkylpolyglycosid als Emulgator und unter Anwendung eines einfachen Blattrührers eine Dispersion von hydriertem Rizinusöl mit einer mittleren Teilchengröße von 70 nm erhalten, welche sich auch nach 6 Monaten Lagerung nicht verändert hatte. Im Lichte des vorgenannten Stands der Technik war es überraschend, daß durch die zunächst als geringfügig erscheinenden Abwandlungen, welche in dem Verfahren gemäß der vorliegenden Erfindung vorgenommen worden waren, die Nachteile des Stands der Technik überwunden werden konnten. Besonders überraschend war es dabei, daß durch eine Vereinfachung der bekannten Lehre, nämlich durch das erfindungsgemäße Ersetzen der Hochdruckhomogenisation durch einfache Rührverfahren verbunden mit dem Eintrag einer deutlich geringeren Rührenergie, bessere Ergebnisse erzielt werden konnten. Diese besseren Ergebnisse bestehen insbesondere in einer erhöhten Lagerstabilität der nanopartikulären Dispersion sowie einer Verminderung der Teilchengröße des nanopartikulären Wachses.

Als weiteres Verfahren zur Herstellung nanopartikulärer Wachse ist das Mahlverfahren zu nennen. Beim Mahlverfahren wird das Wachs durch Mahlen, beispielsweise in einer Schwingmühle, Perlmühle oder Rührwerkskugelmühle zerkleinert, wobei Mahlkörper mit einem mittleren Durchmesser unterhalb von 200 µm verwendet werden. Ggf. erfolgt das Mahlen in Gegenwart eines Oberflächenmodifikationsmittels. Der Mahlvorgang kann trocken oder aber in einem Nicht-Lösungsmittel für das Wachs erfolgen. Der Mahlvorgang kann auch unter Kühlung erfolgen.
Nanopartikel, die bei der Herstellung als Suspension in Wasser oder einer anderen flüssigen Phase anfallen, können durch übliche Verfahren aufkonzentriert bzw. als Feststoff isoliert werden, z. B. durch eine Sprühtrocknung, Gefriertrocknung, oder Ultrafiltration.

Bevorzugt erfolgt die Herstellung der erfindungsgemäß verwendeten nanopartikulären Wachsteilchen nach dem Schmelz-Emulgier-Verfahren oder dem Evaporationsverfahren. Vorzugsweise liegen die Nanopartikel am Ende des Herstellprozesses in Form einer wässrigen oder wasserhaltigen Suspension vor.

Das in der erfindungsgemäßen Verwendung eingesetzte Wachs soll zusammen mit dem verwendeten Oberflächenmodifikationsmittel in der Lage sein, gemäß zumindest einem der vorstehend beschriebenen Herstellverfahren in Wasser stabil dispergierbare Nanopartikel zu ergeben, wobei der mittlere Teilchendurchmesser der Nanopartikel im Bereich von 5 bis 500 nm liegt. Die brauchbaren Wachse und Kombinationen von Wachsen und Oberflächenmodifikationsmitteln lassen sich durch einfache Routineversuche auswählen.

Im Sinne der Erfindung besonders bevorzugt ist die Verwendung von wie eingangs definierten nanopartikulären Wachsen, welche nach einem Verfahren hergestellt werden, in dem
(a) das geschmolzene Wachs in Gegenwart von Wasser mittels eines oder mehrerer Oberflächenmodifikationsmittel emulgiert wird, wobei ganz besonders bevorzugt dabei die Emulgierung ohne intensive Durchmischung, insbesondere ohne Anwendung eines Hochgeschwindigkeitsrührers oder Hochdruckhomogenisators erfolgt, und
(b) die Emulsion unter den Schmelzpunkt der emulgierten Wachströpfchen abgekühlt wird.

Die aufgeführten Herstellverfahren sind lediglich beispielhaft zu verstehen und stellen keine Einschränkung dar.

Nach der Lehre der vorliegenden Erfindung wird ein nanopartikuläres Wachs, welches einen Schmelzpunkt von mindestens 40°C und einen mittleren Teilchendurchmesser im Bereich von 5 bis 500 nm und vorzugsweise im Bereich von 10 bis 200 nm aufweist, in einer kosmetischen und/oder dermatologischen Zubereitung, vorzugsweise einer Zubereitung zur Pflege der Haut und/oder der Nägel eingesetzt. Derartige Zubereitungen sind beispielsweise eine Creme, Lotion, ein Reinigungsprodukt, Deodorant- bzw. Antitranspirant-Stift oder -Creme, eine haarkosmetische Zubereitung wie beispielsweise ein Haarshampoo, Haarkonditionierungsmittel oder ein Haarwachs. Unter den kosmetischen Zubereitungen sind schäumende Zubereitungen wie beispielsweise Duschbäder oder Haarshampoos besonders bevorzugt. Eine weiterhin bevorzugte Zubereitung ist eine Creme, insbesondere eine W/O-Creme.

Als weitere Rezepturbestandteile können in den Zubereitungen im Sinne der Erfindung ein oder mehrere Adjuvantien enthalten sein, wie sie üblicherweise in solchen Zubereitungen verwendet werden, wie z. B. Konservierungsmittel, Bakterizide, Antioxidantien, Parfüme, Schaumbremsen, Farbstoffe, Pigmente, Verdickungsmittel, anfeuchtende und/oder feuchthaltende Substanzen, Tenside, Emulgatoren, Weichmacher, Fette, Öle, Wachse, Silikone, Sequestrierungsmittel, anionische, kationische, nichtionische oder amphotere Polymere, Alkalinisierungs- oder Acidifizierungsmittel, Alkohole, Polyole, Enthärter, Lichtschutzmittel, Elektrolyte und organische Lösungsmittel. Einzelne dieser Stoffe, wie z. B. Emulgatoren, können in den erfindungsgemäßen Zubereitungen einerseits als Adjuvans, unabhängig davon aber auch zusätzlich als Oberflächenmodifikationsmittel der Nanopartikel enthalten sein.

Für den Fall, daß es sich bei der Zubereitung um ein Duschbad handelt, enthält es vorzugsweise
- 0,01 bis 10 Gew.-% eines nanopartikulären Wachses, besonders bevorzugt hydrierten Rizinusöls
- 0 bis 10 Gew.-% eines Rückfettungsmittels, besonders bevorzugt eines oder mehrerer Glyceride, besonders bevorzugt ethoxylierter Glyceride,
- 0,1 bis 10 Gew.-% Acrylsäure-Copolymer
- 1 bis 30 Gew.-% eines oder mehrerer Tenside, vorzugsweise ausgewählt aus der Gruppe, die gebildet wird von anionischen Tensiden, Alkylpolyglycosiden und Betainen
- 0,1 bis 10 Gew.-% eines Polyols, besonders bevorzugt Propylenglykol
- 0,01 bis 2 Gew.-% einer organischen Säure
- 0,01 bis 5 Gew.-% eines Konservierungsmittels und
- 0,01 bis 5 Gew.-% eines Parfüms.

Zur Herstellung der vorstehend genannten Zusammensetzungen wird vorzugsweise eine wäßrige oder wasserhaltige Suspension der Wachs-Nanopartikel mit den weiteren Rezepturbestandteilen in an sich bekannter Weise bei einer Temperatur unterhalb des Schmelzpunkts der Nanopartikel vermischt bzw. dispergiert.

Die Einsatzmenge des nanopartikulären Wachses in der kosmetischen und/oder dermatologischen Zubereitung wird so gewählt, daß die Konzentration des in den Nanopartikeln enthaltenen Wachses - d. h. ohne Berücksichtigung ggf. zusätzlich in den Nanopartikeln enthaltener Oberflächenmodifikationsmittel - zwischen 0,01 und 10, vorzugsweise zwischen 0,1 und 5, und besonders bevorzugt zwischen 0,5 und 2 Gew.-% bezogen auf die Zubereitungen liegt.

Die erfindungsgemäß verwendeten nanopartikulären Wachs-Dispersionen eignen sich in besonderer Weise zum Einsatz in kosmetischen Zusammensetzungen zur Reinigung und/oder Pflege der Haut bzw. der Nägel, ganz besonders bevorzugt für Hautpflegeprodukte wie W/O-Cremes, sowie für Duschbäder.

Bei ihrer Anwendung in Zusammensetzungen zur topischen Behandlung der Haut bewirken die nanopartikulären Wachse gemäß der Erfindung eine Verminderung des Wasserverlustes der Haut und damit eine Erhöhung der Hautfeuchtigkeit, was kosmetisch besonders erwünscht ist. Weiterhin bewirken sie eine Rückfettung der Haut und eine Verbesserung von deren Barriereeigenschaften. Darüber hinaus wird das Hautgefühl verbessert. Es wurde weiterhin gefunden, daß diese Wirkungen der nanopartikulären Wachse gegenüber anderen aus dem Stand der Technik bekannten Wachsformen besonders lange anhalten. Es wird vermutet, daß die Wachs-Nanopartikel nicht nur tief in die Haut eindringen, sondern dort eine Art Depot bilden, welches die kosmetischen und physiologischen Wirkungen des Wachses über einen längeren Zeitraum aufrechterhält. Nicht-nanopartikuläres Wachs verbleibt dagegen an der Oberfläche des Stratum corneum, unterliegt einer leichten Entfernbarkeit z. B. durch mechanische Einwirkungen beim Duschen oder beim Händewaschen, und kann somit keine dauerhaften rückfettenden Eigenschaften auf der Haut erzielen. In einer in-vivo-Studie (Ellenbeugenwaschtest) mit einem Duschbad, welches ein erfindungsgemäßes nanopartikuläres Wachs enthielt, konnte mittels Tesa-Abrissen und analytischer Untersuchungen nachgewiesen werden, daß das erfindungsgemäße nanopartikuläre Wachs in die tieferen Schichten des Stratum corneum penetriert.

Ein weiterer Gegenstand der vorliegenden Erfindung ist die Verwendung eines nanopartikulären Wachses, wobei das Wachs einen Schmelzpunkt von mindestens 40°C hat, der mittlere Teilchendurchmesser des Wachses im Bereich von 5 bis 500 nm und vorzugsweise im Bereich von 10 bis 200 nm liegt, und das Wachs von mindestens einem Oberflächenmodifikationsmittel, das ein Alkylpolyglycosid ist, umhüllt ist, zur Erhöhung der Barrierewirkung und/oder der Erhöhung der Feuchtigkeit der Haut und/oder der Nägel sowie zur Rückfettung der Haut und/oder der Nägel.

Es wurde weiterhin gefunden, daß mittels der erfindungsgemäßen nanopartikulären Wachse die sensorischen Eigenschaften kosmetischer Zusammensetzungen wesentlich verbessert werden können. So weisen W/O-Cremes, welche erfindungsgemäße nanopartikuläre Wachse enthalten, gegenüber analogen Vergleichscremes, in welche die gleiche Menge Wachs in konventioneller Art eingearbeitet worden war, eine deutlich bessere Verteilbarkeit sowie ein schnelleres Einziehvermögen auf der Haut auf. Zudem besitzen sie gegenüber den Vergleichs-Cremes eine sehr viel leichtere Konsistenz, was die Anwendung leichter und für den Anwender angenehmer macht. Sie lassen sich darüber hinaus besser aus Tiegeln von der Art, wie sie häufig für solche Cremes als Behälter verwendet werden, entnehmen und hinterlassen ein angenehmeres und glatteres Hautgefühl.
Ebenso Gegenstand der vorliegenden Erfindung ist die Verwendung eines nanopartikulären Wachses, wobei das Wachs einen Schmelzpunkt von mindestens 40°C hat, der mittlere Teilchendurchmesser des Wachses im Bereich von 5 bis 500 nm und vorzugsweise im Bereich von 10 bis 200 nm liegt, und das Wachs von mindestens einem Oberflächenmodifikationsmittel, das ein Alkylpolyglycosid ist, umhüllt ist, zur Verbesserung der sensorischen Eigenschaften von Zusammensetzungen zur topischen Behandlung der menschlichen Haut, insbesondere von W/O-Cremes.

Unter sensorischen Eigenschaften sind dabei insbesondere folgende zu verstehen: Verteilbarkeit, Einziehvermögen, Konsistenz, Entnahme, Hautglättung und Klebrigkeit. Bezüglich geeigneter Methoden zur Bestimmung dieser Parameter kann auf das Wissen des Fachmanns verwiesen werden.

Weiterhin wurde gefunden, daß die erfindungsgemäßen nanopartikulären Wachs-Dispersionen die Hautverträglichkeit kosmetischer Zusammensetzungen verbessern. Unter der Hautverträglichkeit einer Zusammensetzung ist dabei insbesondere deren Einfluß auf die Hautreaktionen Erythem, Ödem, Schuppung und Fissur bei Applikation der Zusammensetzung auf die Haut zu verstehen.

Ein weiterer Gegenstand der vorliegenden Erfindung ist die Verwendung eines nanopartikulären Wachses, wobei das Wachs einen Schmelzpunkt von mindestens 40°C hat, der mittlere Teilchendurchmesser des Wachses im Bereich von 5 bis 500 nm und vorzugsweise im Bereich von 10 bis 200 nm liegt, und das Wachs von mindestens einem Oberflächenmodifikationsmittel, das ein Alkylpolyglycosid ist, umhüllt ist, zur Verbesserung der Hautverträglichkeit von Zusammensetzungen zur topischen Behandlung der menschlichen Haut.
Ebenfalls Gegenstand der vorliegenden Erfindung ist Verfahren zur pflegenden Behandlung der Haut und/oder der Nägel, bei welchem eine wie vorstehend definierte Zubereitung auf die Haut und/oder die Nägel aufgebracht wird

Die erfindungsgemäßen Wachs-Nanopartikel ermöglichen darüber hinaus die Herstellung von Zusammensetzungen mit einem höheren Wachsanteil, als es mit den aus dem Stand der Technik bekannten Wachsformen möglich ist. Insbesondere ermöglichen sie die Herstellung von schäumenden Formulierungen, die einerseits die gewünschten Effekte, wie z. B. eine gute rückfettende Wirkung, aufweisen und gleichzeitig eine gute Schaumbildung zeigen.

Auf den Nägeln bewirkt das nanopartikuläre Wachs oberflächenaktive Effekte, d. h. der Nagel wird oberflächlich "gewachst". In Nagelpflegepräparaten erzielt man mit der Anwendung von Produkten, welche nanopartikuläres Wachs enthalten, einen Schutz des Nagels vor schädlichen äußeren Einflüssen, wie z.B. der Quellung des Nagels durch tensidhaltige Produkte.

Als weiterer Vorteil kommt hinzu, daß sich die erfindungsgemäßen nanopartikulären Wachse stabil in Formulierungen, selbst wenn sie einen hohen Wasseranteil enthalten, einarbeiten lassen, ohne daß diese instabil werden und ohne daß es zu einer Phasentrennung, Sedimentation oder Teilchengrößenzunahme der Wachspartikel kommt.

Die folgenden Beispiele sollen die vorliegende Erfindung verdeutlichen, ohne sie einzuschränken. Alle Prozentanteile sind, soweit nicht anders angegeben, auf das Gesamtgewicht der Zubereitungen bezogen.

### Beispiele:

### Beispiele 1.1-1.4: Herstellung von Dispersionen nanopartikulärer Wachse

### Beispiel 1.1: Herstellung einer Dispersion von nanopartikulärem hydrierten Rizinusöl

Zu einer auf 98°C erwärmten Schmelze von 3,0 kg Cutina HR (hydriertes Rizinusöl, Warenzeichen der Cognis Deutschland GmbH) wurde eine auf 95°C erwärmte Lösung von 6,0 kg Plantacare 1200 UP (Konservierungsmittel-freies C12-C16-Alkylglycosid mit 51% Aktivsubstanz, Warenzeichen der Cognis Deutschland GmbH) in 21,0 kg dest. Wasser unter Rühren zugegeben. Anschließend wurde das Gemisch eine Stunde bei 95°C nachgerührt, mit 30 g Euxyl K 400 (Methyldibromglutaronitril und Phenoxyethanol, Konservierungsmittel) versetzt und unter Rühren auf Raumtemperatur abgekühlt. Das Rühren erfolgte mit einem Blattrührer bei ca. 50-100 Umdrehungen pro Minute. Es wurden 30,0 kg einer lagerstabilen milchig-weißen Dispersion erhalten, welche nach Bestimmung mittels eines Transmissionselektronenmikroskops Teilchengrößen zwischen 30 und 500 nm aufwies. Bei der Bestimmung der Teilchengröße durch die Laser-Back-Scattering-Methode mittels eines Ultrafine Particle Analyzers (UPA 3.150, Fa. Grimm, Ainring) ergab sich eine mittlere Teilchengröße von 70 nm. In einem Parallelversuch wurde gefunden, daß die Verwendung eines Ankerrührers anstelle eines Blattrührers zum gleichen Versuchsergebnis führt.

### Beispiel 1.2: Herstellung einer Dispersion von nanopartikulärem Cetylstearylalkohol (nicht erfindungsgemäß)

3,0 kg Lanette O (Cetylstearylalkohol, Warenzeichen der Cognis Deutschland GmbH) wurden bei 98°C mit 3,0 kg Texapon N 70 (Natriumlaurylethersulfat mit 2 EO, 70% Aktivsubstanz, Warenzeichen der Cognis Deutschland GmbH) aufgeschmolzen. Die Schmelze wurde zu 24,0 kg auf 98 °C vorgeheiztes Wasser unter Rühren zugegeben. Anschließend wurde das Gemisch eine Stunde bei 98°C nachgerührt, mit 30 g Euxyl K 400 (Methyldibromglutaronitril und Phenoxyethanol, Konservierungsmittel) versetzt und unter Rühren auf Raumtemperatur abgekühlt. Das Rühren erfolgte mit einem Blattrührer bei ca. 50-100 Umdrehungen pro Minute, gleiche Ergebnisse wurden mit einem Ankerrührer anstelle des Blattrührers erhalten. Es wurden 30,0 kg einer lagerstabilen milchig-weißen Dispersion erhalten, welche nach Bestimmung mittels eines Ultrafine Particle Analyzers (UPA 3.150) eine mittlere Teilchengröße von 105 nm aufwies.

### Beispiel 1.3: Herstellung einer Dispersion von nanopartikulärem Cetylstearylalkohol (nicht erfindungsgemäß)

3,0 kg Lanette O (Cetylstearylalkohol, Warenzeichen der Cognis Deutschland GmbH), 3,0 kg Texapon N 70 (Natriumlaurylethersulfat mit 2 EO, Warenzeichen der Cognis Deutschland GmbH) und 15,6 kg dest. Wasser wurden in einem Reaktor vorgelegt und auf 60°C erhitzt. Anschließend wurde das Gemisch unter Rühren weiter auf 90-95°C erhitzt, und bei dieser Temperatur eine halbe Stunde nachgerührt. Das Rühren erfolgte mit einem Blattrührer bei ca. 50-100 Umdrehungen pro Minute, gleiche Ergebnisse wurden mit einem Ankerrührer anstelle des Blattrührers erhalten. Die entstandene Emulsion wurde über eine Düse innerhalb von 15 Minuten in einen zweiten Reaktor entspannt, der durch Evakuieren auf einem Druck zwischen 20 und 30 mbar gehalten wurde, und in welchem 12,0 kg Wasser vorgelegt worden waren. Während der Entspannung wurde die Dispersion von 90-95°C auf 20-25°C abgekühlt. Es wurden 30,5 kg einer lagerstabilen Suspension erhalten, welche eine mittlere Teilchengröße von 200 nm aufwies.

### Beispiel 1.4: Herstellung einer Dispersion von nanopartikulärem Cetylstearylalkohol (nicht erfindungsgemäß)

3,0 kg Lanette O (Cetylstearylalkohol, Warenzeichen der Cognis Deutschland GmbH), 3,0 kg Eumulgin HRE 455 (gehärtetes Rizinusöl mit 40 EO in Propylenglykol/Wasser, Warenzeichen der Cognis Deutschland GmbH) und 33,1 kg dest. Wasser wurden in einem Reaktor vorgelegt und auf 60°C erhitzt. Anschließend wurde das Gemisch unter Rühren weiter auf 90-95°C erhitzt, und bei dieser Temperatur eine halbe Stunde nachgerührt. Das Rühren erfolgte mit einem Blattrührer bei ca. 50-100 Umdrehungen pro Minute, gleiche Ergebnisse wurden mit einem Ankerrührer anstelle des Blattrührers erhalten. Die entstandene Emulsion wurde über eine Düse innerhalb von 15 Minuten in einen zweiten Reaktor entspannt, der durch Evakuieren auf einem Druck zwischen 20 und 30 mbar gehalten wurde. Während der Entspannung wurde die Dispersion von 90-95°C auf 20-25°C abgekühlt. Es wurden 32,0 kg einer lagerstabilen Suspension erhalten, welche eine mittlere Teilchengröße von 200 nm aufwies.

### Beispiele 2.1 (Vergleichsbeispiel) und 2.2 (erfindungsgemäßes Beispiel):

### Duschbäder:

| | | Beispiel 2.1 | Beispiel 2.2 |
|---|---|---|---|
| Rezepturkomponente | | Gew.-% | Gew.-% |
| Texapon | Natriumlaurylethersulfat+2EO 70 % AS | 15 | 15 |
| Plantacare 1200 | APG mit 51 % AS (AS = Aktivsubstanz) | 2 | - |
| Rewoteric | Dinatrium Cocoamphodiacetat | 1,55 | 0,55 |
| Tego Betain F50 | Kokofettsäureamido-propylbetain 38 % AS (7 % NaCl) | 5 | 5 |
| Natrium-Benzoat | 100 %ig | 0,4 | 0,4 |
| Parfüm | 100 %ig | 1,2 | 1,2 |
| Cetiol HE | PEG-7 Glyceryl Cocoate | 0,5 | 0,5 |
| Dispersion aus Beispiel 1.1 | 10 % Cutina HR 20 % Plantacare 1200 0,1 % Euxyl K400 ad 100 % Wasser (Gew.-% bezogen auf das Gesamtgewicht der Dispersion) | - | 10 |
| Wasser | Vollentsalztes Wasser | ad 100 | ad 100 |

Das erfindungsgemäße Duschbad 2.2 enthielt 1 Gew.-% Cutina HR in nanopartikulärer Form, bezogen auf das Gesamtgewicht der Zubereitung.

### Beispiel 3: Stabilitätsversuche mit Duschbädern

Zusätzlich zu dem unter Beispiel 2.2 aufgeführten Duschbad wurden analoge Duschbäder hergestellt, in welchen unter Beibehaltung von Art und Konzentration der übrigen Wirkstoffe jeweils nur die Einsatzkonzentration der Dispersion aus Beispiel 1.1 auf 2, 5 bzw. 20 Gew.-% abgeändert und jeweils ad 100 Gew.-% mit Wasser aufgefüllt wurde, so daß Duschbäder mit einer Konzentration von 0,2, 0,5, bzw. 2 Gew.-% Cutina HR in nanopartikulärer Form erhalten wurden. Um die Stabilität der Duschbäder mit den eingearbeiteten Cutina HR-Nanopartikeln zu untersuchen, wurden sie über einen Zeitraum von vier Monaten einer optischen makroskopischen Analyse mit einem TurbiScan MA 1000 der Fa. Particle Metrix Instruments GmbH, Herrsching, unterzogen. Zur Analyse wurden die Proben in einer Glasküvette mit einem Durchmesser von 12 mm mit Licht der Wellenlänge λ=850nm durchstrahlt und die Parameter Rückstreuung sowie Transmission ermittelt. Diese Messungen wurden mit Duschbädern mit einer Konzentration von 0,2, 0,5, 1 bzw. 2 Gew.-% Cutina HR in nanopartikulärer Form bei 5 °C, Raumtemperatur und 45 °C durchgeführt. Nach vier Monaten Lagerung bei 45 °C zeigten die Proben im TurbiScan lediglich geringfügige Schwankungen in der Rückstreuung. So betrugen beispielsweise die Schwankungen in der Rückstreuung der Probe mit 1 Gew.-% Cutina HR-Nanopartikeln im TurbiScan lediglich 0,99 bis 1,73 % des Ausgangswertes. Über die gesamte Länge der Meßküvette zeigten die Duschbäder eine homogene Rückstreuung und Transmission und damit keine signifikanten Instabilitäten.

### Beispiel 4: In-vivo-Tests der kosmetischen Eigenschaften der Duschbäder aus Beispiel 2 an Probanden: Ellenbeugenwaschtest mit einer 10 Gew.-%igen wäßrigen Lösung des Duschbads aus Beispiel 2.2

20 Probanden wuschen sich über einen Zeitraum von zwei Wochen jeweils zweimal täglich unter kontrollierten Bedingungen die Ellenbeugen des linken bzw. rechten Unterarms mit einer 10 Gew.-%igen wäßrigen Lösung des Duschbads aus Vergleichsbeispiel 2.1 bzw. des erfindungsgemäßem Beispiels 2.2. Vor und nach jeder Waschung wurden objektive Reaktionen der Haut (Erytheme, Ödeme, Schuppungen, Fissuren) sowie subjektive Reaktionen (Brennen, Jucken) festgehalten. Vor Beginn und am Ende der Studie wurde der transepidermale Wasserverlust (TEWL) der Haut bestimmt. Im Anschluß an die Studie wurde von den Probanden ein Fragebogen ausgefüllt, sowie von jedem Arm 6 Tesastripps zur Analytik auf Cutina HR abgenommen.Die visuelle Ablesung der Hautreaktionen ergab jeweils nur sehr geringe Reaktionen der Haut auf das Vergleichs-Duschbad 2.1 oder das erfindungsgemäße Duschbad 2.2. Die Hautfeuchtigkeit wurde vor Beginn der Studie sowie vor der letzten Waschung und 4 Stunden nach der letzten Waschung gemessen. Der TEWL-Wert war nach Anwendung des erfindungsgemäßen Duschbads 2.2 geringer als nach Anwendung des Vergleichs-Duschbads 2.1. Am Ende der Studie wurde bei den Probanden das Hautgefühl nach der Anwendung der beiden Duschbad-Lösungen abgefragt. Bei dem erfindungsgemäßen Duschbad 2.2 zeigten sich relativ zu dem Vergleichs-Duschbad 2.1 Tendenzen in Richtung eines gecremten Hautgefühls. Weiterhin wurde die Haut nach Anwendung des erfindungsgemäßen Duschbads von 10 % der Probanden als fettiger empfunden, während das bei dem Vergleichs-Duschbad von keinem Probanden so empfunden wurde. Zur Objektivierung des rückfettenden Effektes der angewandten Formulierungen wurde der Cutina-HR-Gehalt der oberen Stratumcorneum-Schichten der Haut nach Anwendung der Duschbad-Lösungen analytisch bestimmt. Hierzu wurde von jedem Unterarm ein Tesafilm-Stripping durchgeführt. Ein 10 cm langes und 0,9 cm breites Stück Tesafilm wurde auf das behandelte bzw. unbehandelte Hautareal aufgebracht, leicht angedrückt und dann wieder entfernt. Dieser Vorgang wurde an der gleichen Stelle insgesamt 6 mal wiederholt. Jeweils die ersten drei sowie die letzten drei Tesastreifen wurden gepoolt und analytisch der Gehalt an hydriertem Rizinusöl bestimmt. Während bei dem Vergleichs-Duschbad in den ersten drei Tesastripps durchschnittlich nur 1,61 µg hydriertes Rizinusöl gefunden wurden, betrug der Gehalt der ersten drei Tesatripps nach Anwendung des erfindungsgemäßen Duschbads durchschnittlich 4,96 µg hydriertes Rizinusöl. In den Stripps 4 bis 6 wurden 0,98 (Vergleichs-Duschbad) bzw. 1,85 µg (erfindungsgemäßes Duschbad) hydriertes Rizinusöl nachgewiesen. Somit belegen die Ergebnisse eine deutliche Einlagerung von Cutina HR in das Stratum Corneum durch die Anwendung der erfindungsgemäßen Duschbad-Lösung.

### Beispiele 5.1 (Vergleichsbeispiel) und 5.2 (erfindungsgemäßes Beispiel): W/O-Cremes

| | | Beispiel 5.1 | Beispiel 5.2 |
|---|---|---|---|
| Rezepturkomponente | INCI-Bezeichnung | Gew.-% | Gew.-% |
| Lameform TGI | Polyglyceryl-3 Diisostearat | 4.0 | 4.0 |
| Monomuls 90-018 | Glycerylmonooleat | 2.0 | 2.0 |
| Bienenwachs | Beeswax | 7.0 | 7.0 |
| Cetiol OE | Di-n-octylether | 10.0 | 10.0 |
| Cetiol PGL | Hexyldecanol/Hexyldecyl Laurat | 10.0 | 10.0 |
| Cutina HR | Hydrogenated Castor Oil | 1.0 | - |
| Glycerin (86%ig) | | 5.0 | 5.0 |
| Mg-Sulfat 7H2O | | 1.0 | 1.0 |
| Plantacare 1200 UP | APG 1200 (50 % Aktivsubstanz) | 2.0 | - |
| Phenonip | | 0.3 | 0.3 |
| Dispersion aus Beispiel 1.1 | | - | 10.0 |
| Wasser (vollentsalzt) | | ad 100.0 | ad 100.0 |

### Beispiel 6: Sensory Assessment mit den W/O-Cremes aus Beispielen 5.1 und 5.2

Die erfindungsgemäße W/O-Creme 5.2 zeigte gegenüber dem Vergleichsbeispiel 5.1 eine deutlich bessere Verteilbarkeit und ein schnelleres Einziehvermögen auf der Haut. Zudem besaß sie eine sehr viel leichtere Konsistenz, welche die Anwendung leichter und für den Anwender angenehmer machte. Sie ließ sich darüber hinaus besser aus einem Tiegel von der Art, wie er für solche Cremes üblicherweise als Behälter eingesetzt wird, entnehmen und hinterließ ein glatteres Hautgefühl.

### Beispiele 7.1 - 7.7: Weitere Zubereitungen mit nanopartikulären Wachsen

### 7.1 und 7.2 Gesichtsreiniger:

| | Beispiel 7.1 | Beispiel 7.2 nicht erfindungsgemäß |
|---|---|---|
| Rezepturkomponente | Gew.-% | Gew.-% |
| Glycerin | 2,0 | 2,0 |
| Acrylate/C10-30 Alkylacrylat Crosspolymer | 0,9 | 0,9 |
| Triethanolamin | 1,1 | 1,1 |
| Cocoamidopropylbetain | 5,0 | 5,0 |
| Na-Methylcocoyltaurat | 8,0 | 8,0 |
| Dispersion aus Beispiel 1.1 | 20,0 | 0 |
| Dispersion aus Beispiel 1.2 | 0 | 20,0 |
| Formalin | 0,08 | 0,08 |
| Wasser | ad 100 | ad 100 |

### 7.3 und 7.4: W/O-Cremes

| | Beispiel 7.3 | Beispiel 7.4 nicht erfindungsgemäß |
|---|---|---|
| Rezepturkomponente | Gew.-% | Gew.-% |
| Dicaprylylether | 7,0 | 7,0 |
| Decyloleat | 7,5 | 7,5 |
| Dispersion aus Beispiel 1.1 | 30 | 0 |
| Dispersion aus Beispiel 1.2 | 0 | 30 |
| Vitamin E | 2,0 | 2,0 |
| Glycerin | 5,0 | 5,0 |
| Magnesiumsulfat | 0,7 | 0,7 |
| Formalin (37%ig) | 0,08 | 0,08 |
| Wasser | ad 100 | ad 100 |

### 7.5: Deo-Roll-on (nicht erfindungsgemäß)

| Rezepturkomponente | Gew.-% |
|---|---|
| Ethanol 96%ig | 30 |
| Eumulgin B1 | 2,0 |
| Eumulgin B3 | 2,0 |
| Locron L | 16 |
| Natrosol 250HR 2%ig in Wasser | 22,5 |
| Parfümöl | 0,5 |
| Dispersion aus Beispiel 1.2 | 2,0 |
| Wasser dest. | ad 100 |

### 7.6 und 7.7: Duschbäder

| | Beispiel 7.6 (Vergleichsbeispiel) | Beispiel 7.7 |
|---|---|---|
| Rezepturkomponente | Gew.-% | Gew.-% |
| Cetiol HE | 1,0 | 1,0 |
| Merquat 280 , | 1,5 | 1,5 |
| Texapon N70 | 12,5 | 12,5 |
| Plantacare 1200 UP | 2,0 | 0 |
| Propylene Glycol | 2,0 | 2,0 |
| Lactic Acid | 0,1 | 0,1 |
| Sodium Benzoate | 0,4 | 0,4 |
| Dispersion aus Beispiel 1.1 | 0 | 10 |
| Sodium Lactate | 0,06 | 0,06 |
| Parfüm | 0,3 | 0,3 |
| Wasser | ad 100 | ad 100 |

Die Hautverträglichkeit der Duschbäder aus den Beispielen 7.6 und 7.7 wurde in einem 24-Stunden-Patch-Test geprüft, welcher in Anlehnung an die COLIPA-Richtlinien durchgeführt wurde. Hierzu wurden an 20 Probanden die Pflaster (Finn Chambers) okklusiv für 24 h auf dem Rücken appliziert. Nach 6, 24, 48 und 72 Stunden wurden die Hautreaktionen Erythem, Ödem, Schuppung und Fissur abgelesen. Score-Werte wurden zu jedem Ablesezeitpunkt, nach der Stärke der Reaktion gewichtet, ermittelt und durch die Probandenzahl geteilt. Score-Werte wurden für die Parameter Erythem, Ödem, Schuppung und Fissur ermittelt (nach Frosch, P. J. and Kligman, A. M., 1979. J. Am. Acad. Dermatol. 1, 35-41). Die Ergebnisse sind in der nachfolgenden Tabelle aufgeführt.

| | Summe Erytheme | Summe Erytheme, Ödeme, Schuppung, Fissur | Bemerkungen |
|---|---|---|---|
| Vergleichsbeispiel 7.6 | 2,25 | 4,55 | |
| Erfindungsgemäßes Beispiel 7.7 | 1,90 | 3,70 | Signifikant besser bzgl. Schuppung |

Das erfindungsgemäße Beispiel 7.7 wies gegenüber dem Vergleichsbeispiel 7.6 eine signifikante Verminderung des Reizsummenscores auf. Dies belegt, daß der Zusatz der erfindungsgemäßen Dispersion von nanopartikulärem Cutina HR zu der Duschbadrezeptur des Vergleichsbeispiels deren Hautverträglichkeit signifikant verbesserte.

## Patentansprüche

1. Nanopartikuläres Wachs, wobei das Wachs einen Schmelzpunkt von mindestens 40°C hat und der mittlere Teilchendurchmesser des Wachses im Bereich von 5 bis 500 nm und vorzugsweise im Bereich von 10 bis 200 nm liegt, **dadurch gekennzeichnet, dass** das nanopartikuläre Wachs von mindestens einem Oberflächenmodifikationsmittel, das ein Alkylpolyglycosid ist, umhüllt ist.

2. Nanopartikuläres Wachs gemäß Anspruch 1, **dadurch gekennzeichnet, dass** das Wachs ausgewählt ist aus der Gruppe, die gebildet wird von natürlichen Wachsen, chemisch modifizierten Wachsen, synthetischen Wachsen, Fettalkoholen mit 14 bis 40 Kohlenstoffatomen und Wachsestem mit 20 bis 44 Kohlenstoffatomen.

3. Nanopartikuläres Wachs gemäß Anspruch 1 oder 2, **dadurch gekennzeichnet, dass** das Wachs gehärtetes Rizinusöl oder Cetylstearylalkohol ist.

4. Nanopartikuläres Wachs gemäß einem der Ansprüche 1 - 3, **dadurch gekennzeichnet, dass** für die Herstellung des nanopartikulären Wachses das Wachs und das Oberflächenmodifikationsmittel im Gewichtsverhältnis 1 : 50 bis 50 : 1, vorzugsweise 1 : 5 bis 10 : 1 und insbesondere 1 : 2 bis 2 : 1, eingesetzt werden.

5. Nanopartikuläres Wachs gemäß Anspruch 4, **dadurch gekennzeichnet, dass** ein Gemisch aus zwei oder mehr Oberflächenmodifikationsmitteln eingesetzt wird.

6. Nanopartikuläres Wachs gemäß einem der Ansprüche 1-5, **dadurch gekennzeichnet, dass** es in Form einer wässrigen oder wasserhaltigen Suspension vorliegt.

7. Nanopartikuläres Wachs gemäß einem der Ansprüche 1 - 6, **dadurch gekennzeichnet, dass** der Alkylrest des Alkylpolyglycosids zwischen 12 und 16 Kohlenstoffatome enthält.

8. Kosmetische oder dermatologische Zubereitung, enthaltend ein oder mehrere übliche Adjuvantien, ausgewählt aus Konservierungsmitteln, Bakteriziden, Antioxidantien, Parfüms, Schaumbremsen, Farbstoffen, Pigmenten, Verdickungsmitteln, anfeuchtenden und/oder feuchthaltenden Substanzen, Tensiden, Emulgatoren, Weichmachern, Fetten, Ölen, Wachsen, Silikonen, Sequestrierungsmitteln, anionischen, kationischen, nichtionischen oder amphoteren Polymeren, Alkalinisierungs- oder Acidifizierungsmitteln, Alkoholen, Polyolen, Enthärtern, Lichtschutzmitteln, Elektrolyten und organischen Lösungsmitteln, **dadurch gekennzeichnet, dass** ein nanopartikuläres Wachs gemäß einem der Ansprüche 1 - 7 enthalten ist.

9. Kosmetische oder dermatologische Zubereitung gemäß Anspruch 8, **dadurch gekennzeichnet, dass** das nanopartikuläre Wachs in der Zubereitung in einer Konzentration von 0,01 bis 10 und vorzugsweise von 0,1 bis 5 Gew.-%, bezogen auf das Gesamtgewicht der Zubereitung, enthalten ist.

10. Kosmetische oder dermatologische Zubereitung gemäß Anspruch 8 oder 9, **dadurch gekennzeichnet, dass** die Zubereitung eine Creme, Lotion, ein Reinigungsprodukt, Deodorant- bzw. Antitranspirant-Stift oder -Creme, eine haarkosmetische Zubereitung oder eine schäumende Zubereitung ist.

11. Nicht-therapeutische, kosmetische Verwendung eines nanopartikulären Wachses gemäß einem der Ansprüche 1 - 7 oder einer Zubereitung gemäß einem der Ansprüche 8 - 10 zur Reinigung und/oder Pflege der Haut und/oder der Nägel.

12. Nicht-therapeutische, kosmetische Verwendung gemäß Anspruch 11 zur Erhöhung der Barrierewirkung und/oder zur Erhöhung der Feuchtigkeit und/oder zur Rückfettung der Haut und/oder der Nägel.

13. Verwendung eines nanopartikulären Wachses gemäß einem der Ansprüche 1 - 7 zur Verbesserung der sensorischen Eigenschaften von Zubereitungen zur topischen Behandlung der menschlichen Haut.

14. Verwendung eines nanopartikulären Wachses gemäß einem der Ansprüche 1 7 zur Verbesserung der Hautverträglichkeit von. Zubereitungen zur topischen Behandlung der menschlichen Haut.

15. Nicht-therapeutisches, kosmetisches Verfahren zur pflegenden Behandlung der Haut und/oder der Nägel, **dadurch gekennzeichnet, dass** ein nanopartikuläres Wachs gemäß einem der Ansprüche 1 - 7 oder eine Zubereitung gemäß einem der Ansprüche 8 - 10 auf die Haut und/oder die Nägel aufgebracht wird.

## Claims

1. Nanoparticulate wax, where the wax has a melting point of at least 40°C and the average particle diameter of the wax is in the range from 5 to 500 nm and preferably in the range from 10 to 200 nm, **characterized in that** the nanoparticulate wax is surrounded by at least one surface modifier which is an alkyl polyglycoside.

2. Nanoparticulate wax according to Claim 1, **characterized in that** the wax is chosen from the group which is formed from natural waxes, chemically modified waxes, synthetic waxes, fatty alcohols having 14 to 40 carbon atoms and wax esters having 20 to 44 carbon atoms.

3. Nanoparticulate wax according to Claim 1 or 2, **characterized in that** the wax is hydrogenated castor oil or cetylstearyl alcohol.

4. Nanoparticulate wax according to one of Claims 1-3, **characterized in that** the wax and the surface modifier are used in the weight ratio 1:50 to 50:1, preferably 1:5 to 10:1 and in particular 1:2 to 2:1, for the preparation of the nanoparticulate wax.

5. Nanoparticulate wax according to Claim 4, **characterized in that** a mixture of two or more surface modifiers is used.

6. Nanoparticulate wax according to one of Claims 1-5, **characterized in that** it is in the form of an aqueous or water-containing suspension.

7. Nanoparticulate wax according to one of Claims 1-6, **characterized in that** the alkyl radical of the alkyl polyglycoside contains between 12 and 16 carbon atoms.

8. Cosmetic or dermatological preparation comprising one or more customary adjuvants chosen from preservatives, bactericides, antioxidants, perfumes, antifoams, dyes, pigments, thickeners, moisturizing and/or humectant substances, surfactants, emulsifiers, emollients, fats, oils, waxes, silicones, sequestrants, anionic, cationic, nonionic or amphoteric polymers, alkalinizing or acidifying agents, alcohols, polyols, softeners, photoprotective agents, electrolytes and organic solvents, **characterized in that** a nanoparticulate wax according to one of Claims 1-7 is present.

9. Cosmetic or dermatological preparation according to Claim 8, **characterized in that** the nanoparticulate wax is present in the preparation in a concentration of from 0.01 to 10% by weight and preferably from 0.1 to 5% by weight, based on the total weight of the preparation.

10. Cosmetic or dermatological preparation according to Claim 8 or 9, **characterized in that** the preparation is a cream, lotion, cleansing product, deodorant and/or antiperspirant stick or cream, a hair cosmetic preparation or a foaming preparation.

11. Non-therapeutic, cosmetic use of a nanoparticulate wax according to one of Claims 1-7 or of a preparation according to one of Claims 8-10 for the cleansing and/or care of the skin and/or the nails.

12. Non-therapeutic, cosmetic use according to Claim 11 for increasing the barrier effect and/or for increasing the moisture and/or for refatting the skin and/or the nails.

13. Use of a nanoparticulate wax according to one of Claims 1-7 for improving the sensory properties of preparations for the topical treatment of human skin.

14. Use of a nanoparticulate wax according to one of Claims 1-7 for improving the skin compatibility of preparations for the topical treatment of human skin.

15. Non-therapeutic, cosmetic method for the caring treatment of the skin and/or the nails, **characterized in that** a nanoparticulate wax according to one of Claims 1-7 or a preparation according to one of Claims 8-10 is applied to the skin and/or the nails.

## Revendications

1. Cire nanoparticulaire, la cire possédant un point de fusion s'élevant à au moins 40 °C et le diamètre moyen de particule de la cire se situant dans la plage de 5 à 500 nm, de préférence dans la plage de 10 à 200 nm, **caractérisée en ce que** la cire nanoparticulaire est enveloppée d'au moins un agent modificateur de surface qui représente un alkylpolyglycoside.

2. Cire nanoparticulaire selon la revendication 1, **caractérisée en ce que** la cire est choisie parmi le groupe qui est formé par des cires naturelles, des cires chimiquement modifiées, des cires synthétiques, des alcools gras contenant de 14 à 40 atomes de carbone et des esters de cires contenant de 20 à 44 atomes de carbone.

3. Cire nanoparticulaire selon la revendication 1 ou 2, **caractérisée en ce que** la cire représente de l'huile de ricin durcie ou de l'alcool cétylstéarylique.

4. Cire nanoparticulaire selon l'une quelconque des revendications 1 à 3, **caractérisée en ce qu'**on met en oeuvre, pour la préparation de la cire nanoparticulaire, la cire et l'agent modificateur de surface dans le rapport pondéral de 1 : 50 à 50 : 1, de préférence de 1 : 5 à 10: 1 et en particulier de 1 : 2 à 2 : 1.

5. Cire nanoparticulaire selon la revendication 4, **caractérisée en ce qu'**on met en oeuvre un mélange de deux agents modificateurs de surface ou plus.

6. Cire nanoparticulaire selon l'une quelconque des revendications 1 à 5, **caractérisée en ce qu'**elle est présente sous la forme d'une suspension aqueuse ou contenant de l'eau:

7. Cire nanoparticulaire selon l'une quelconque des revendications 1 à 6, **caractérisée en ce que** le radical alkyle de l'alkylpolyglycoside contient entre 12 et 16 atomes de carbone.

8. Préparation cosmétique ou dermatologique, contenant un ou plusieurs adjuvants habituels choisis parmi le groupe comprenant des conservateurs, des bactéricides, des antioxydants, des parfums, des agents antimousse, des colorants, des pigments, des épaississants, des substances légèrement humidifiantes et/ou qui gardent l'humidité, des agents tensioactifs, des émulsifiants, des plastifiants, des graisses, des huiles, des cires, des silicones, des agents de séquestration, des polymères anioniques, cationiques, non ioniques ou amphotères, des agents d'alcalinisation ou d'acidification, des alcools, des polyols, des adoucisseurs, des agents de protection contre l'effet de la lumière, des électrolytes et des solvants organiques, **caractérisée en ce qu'**elle contient une cire nanoparticulaire selon l'une quelconque des revendications 1 à 7.

9. Préparation cosmétique ou dermatologique selon la revendication 8, **caractérisée en ce que** la cire nanoparticulaire est contenue dans la préparation en une concentration de 0,01 à 10, et de préférence de 0,1 à 5 % en poids, rapportés au poids total de la préparation.

10. Préparation cosmétique ou dermatologique selon la revendication 8 ou 9, **caractérisée en ce que** la préparation est une crème, une lotion, un produit de nettoyage, un déodorant, respectivement un antitranspirant sous forme de bâton ou sous forme de crème, une préparation cosmétique capillaire ou encore une préparation moussante.

11. Utilisation cosmétique non thérapeutique d'une cire nanoparticulaire selon l'une quelconque des revendications 1 à 7 ou d'une préparation selon l'une quelconque des revendications 8 à 10 pour le nettoyage et/ou le soin de la peau et/ou des ongles.

12. Utilisation cosmétique non thérapeutique selon la revendication 11 pour augmenter l'effet de barrière et/ou pour augmenter l'humidité et/ou pour le regraissage de la peau et/ou des ongles.

13. Utilisation d'une cire nanoparticulaire selon l'une quelconque des revendications 1 à 7 pour améliorer les propriétés sensorielles de préparations pour le traitement local de la peau humaine.

14. Utilisation d'une cire nanoparticulaire selon l'une quelconque des revendications 1 à 7 pour améliorer la compatibilité cutanée de préparations pour le traitement local de la peau humaine.

15. Procédé cosmétique non thérapeutique pour le traitement de soins de la peau et/ou des ongles, **caractérisé en ce qu'**on applique une cire nanoparticulaire selon l'une quelconque des revendications 1 à 7 ou une préparation selon l'une quelconque des revendications 8 à 10 sur la peau et/ou sur les ongles.
